# EUROPEAN PATENT APPLICATION

(11) **EP 4 368 237 A1**
(43) Date of publication of application: **15.05.2024**
(21) Application number: 22206041.0
(22) Date of filing: 08.11.2022
(51) Int. Cl.: A61N 1/05, A61N 1/375, A61N 1/362, A61N 1/39

(54) **IMPLANTABLE LEAD AND MANUFACTURING METHOD THEREFOR**

(71) Applicant: BIOTRONIK SE & Co. KG, 12359 Berlin (DE)
(72) Inventor: Trip, Erik, 59200 Kuala Lumpur (MY); Kolberg, Gernot, 12161 Berlin (DE); Zedler, Stephan, 12309 Berlin (DE); Palm, Jochen, 15831 Mahlow (DE); Grauhan, Ole, 14163 Berlin (DE); Dohmen, Daniel, San Francisco, 94107 (US)
(74) Representative: Biotronik Corporate Services SE

(57) **Abstract**

The invention refers to an implantable lead (10, 416, 417) of the Bisping type extending between a proximal end and a distal end and having an inner member (11, 220, 320), an outer tubular member (12), a terminal pin (110) at the proximal end and a fixation helix (190, 418, 422) at the distal end, wherein the outer tubular member comprises an electric conductor (140, 230) and the inner member is arranged between the terminal pin and the fixation helix, wherein the inner member comprises an electrically conducting wire spiral (150). In order to improve torque transmission from the proximal to the distal end of the lead the conducting wire spiral (150) is fully covered at its exterior surface by an electrically insulating layer (160, 220, 320) formed by extrusion onto the wire spiral. The invention further refers to a respective manufacturing method of such implantable lead and a system comprising at least one of such implantable lead and a control device (e.g. a pacemaker).

## Description

The present invention relates to an implantable lead extending between a proximal end and a distal end and having an inner member, an outer tubular member, a terminal pin at the proximal end and a fixation helix at the distal end, wherein the outer tubular member comprises an electric conductor and the inner member is arranged between the terminal pin and the fixation helix. The implantable lead is of the type where the inner member comprises an electrically conducting wire spiral. The inner member is accommodated within the outer tubular member. The invention is further directed to a manufacturing method for such implantable lead. Furthermore, an implantable cardiac system comprising such implantable lead is described.

The present invention relates to a lead (also referred to as electrode or electrode lead) for implantation into the heart, particularly for stimulating the heart muscle, the lead being of a type which includes a screw-like protruding fixation helix at the distal end of the lead for screwing into cardiac tissue. This lead type is also called Bisping electrode. Such lead serves to transmit stimulation pulses and/or physiological signals between the heart muscle and a likewise implanted control device that may comprise a pulse generator. The control device connected to the lead is also referred to as artificial cardiac pacemaker, cardiac defibrillator or implantable cardiac monitor.

The electrically conducting wire spiral of the inner member transmits or communicates the stimulation pulses and/or physiological (electric) signals between the heart and the control device. Usually, the wire spiral comprises three to six filaments of round or flat wire that are electrically insulated with regard to the surrounding outer member by a silicone sleeve. For that, the wire spiral is electrically connected to the terminal pin at the proximal end and to the fixation helix at the distal end of the lead, wherein the electrical connection may comprise a direct and an indirect electrical connection. The electric conductor of the outer tubular member surrounding the inner member is electrically insulated with regard to the wire spiral of the inner member.

To fix the electrode mechanically within the heart's muscle and to provide an electrical connection to the tissue, the fixation helix is screwed from the distal end of the lead into the heart's tissue at a pre-defined location of the heart. The rotational and translational movement of the fixation helix is usually provided by rotation of the terminal pin and torque transmission mainly via the wire spiral. It is observed that such torque transmission from the terminal pin to the fixation helix is affected by elasticity of the wire spiral and the outer member in circumferential direction (e.g. twisting) as well as by friction between the inner member and the outer member leading to poor control of fixation helix movement during penetration the heart's muscle. Additionally, because of these properties of the inner member a great number of rotations from the distal end of the lead is needed to provide full protrusion of the fixation helix into the pre-determined tissue.

A further disadvantage of prior art leads is that inserting the wire spiral into the silicone sleeve is cost and time intensive and not automatable because the silicone sleeve produces high friction at its inner surface and because it appears to be very flexible (i.e. has a very low stiffness).

Accordingly, it is an objective of the present invention to improve torque transmission from the pin to the fixation helix and to provide a more cost-effective manufacturing method.

The above problem is solved by a manufacturing method having the features of claim 1, an implantable lead having the features of claim 7 and by an implantable cardiac system with the features of claim 13.

In particular, the above problem is solved by a manufacturing method for an implantable lead extending between a proximal end and a distal end and having an inner member, an outer tubular member, a terminal pin at the proximal end and a fixation helix at the distal end. Further, the outer tubular member comprises an electric conductor and the inner member is arranged between the terminal pin and the fixation helix. The inner member comprises an electrically conducting wire spiral. The manufacturing method comprises the following steps:
providing the electrically conducting wire spiral and extruding an electrically insulating layer onto the conducting wire spiral and afterwards inserting the inner member into the outer tubular member. Carrying out of further steps between the extrusion step and the inserting step are possible.

The inner member comprises the electrically conducting wire spiral that may comprise, for example, 6 to 8 filaments, wherein each filament is formed by a round or flat wire. The filaments are electrically insulated against each other but the whole wire spiral is electrically insulated and mechanically protected against the electric conductor of the outer member by the electrically insulating layer (non-conducting layer) that is extruded onto the exterior surface of the wire spiral. The electrically insulating layer covers this wire spiral over its entire length and its full exterior surface (i.e. the surface forming the outer shell surface of the wire spiral). Herein, the length is the dimension of the respective part or element of the lead along its longitudinal extension. During the extrusion process, the material of the electrically insulating layer may also fill the space between neighboring windings of the wire spiral so that the completely extruded electrically conducting layer forms a continuous surface. Accordingly, the electrically insulating layer forms a positive-locking connection with the wire spiral.

The outer member may comprise a second wire spiral as an electric conductor and/or at least one straight electric conductor embedded within or covered by an electrically insulating sleeve (non-conducting sleeve), wherein the axis of the wire spiral or the straight electric conductor may extend parallel to the longitudinal direction of the inner member at least part of the full length of the outer member. The inner lumen of the tubular outer member is provided such that the inner member may be inserted into this lumen giving a light press fit to the inner member when inserted into the outer member, wherein the inner member may be rotatable and longitudinally movable within the outer member to allow screwing of the fixation helix.

The extrusion process may be provided using a nozzle, in particular a structured nozzle, and/or any other tool, and/or at a temperature between 5 and 40 degrees Celsius, in particular 18 degrees Celsius. The material of the extruded layer may be a thermoplastic material, the material may comprise at least one material selected from the group comprising polyurethanes (PU), polyester-urethanes (PEU), polyether-urethanes (PEEU), polycarbonate-urethanes (PCU), silicone based polycarbonate-urethanes (PCU), polycarbonate-polyurea-urethanes (PCHU), polydimethylsiloxane-urethanes (PSU), polyisobutylene-urethanes (PIU), polyisobutylene based copolymers (PIC), polyether-block amides (PEBA, for example PEBAX), polyimides (PI), fluorinated hydrocarbons, ethylene tetrafluoroethylene copolymers (ETFE), polytetrafluoroethylenes (PTFE), polysulfones (PSU), polyethylenes (PE), polypropylenes (PP), polyamides (PA), and polyether ether ketones (PEEK). The thickness of the electrically insulating layer depends on the material and may be, for example between 0,05 and 0,2 mm, preferably 0,1 mm, wherein the thickness is measured as a maximum thickness value in radial direction of the inner member and from the exterior surface of the wire spiral of the inner member.

After extrusion and subsequent cooling the inner member comprising the wire spiral and the electrically insulating layer extruded onto the exterior surface of the wire spiral over its entire length and surface area (and, if applicable, after some additional steps) is inserted into the inner lumen of the tubular outer member forming a light press-fit as indicated above.

By extrusion, the electrically insulating layer is permanently mechanically linked (by positive-locking and/or firm bonding to the wire spiral and the thermoplastic material has low elasticity such that it does not absorb and dampen a torque that is provided at the proximal end to the inner member but directly transmits the torque to the distal end comprising the fixation helix. That means that by the extrusion process the insulating layer is fixed non-rotatably to the wire spiral. Accordingly, the rotational and translational movement of the fixation helix may be more precisely controlled by the user rotating the terminal pin fixed at the proximal end of the lead. Additionally, the extrusion stop allows automation of the manufacturing steps creating the inner member.

In one embodiment of the manufacturing method, the extrusion is provided such that a profile is created at the outer surface of the electrically insulating layer. The profile may reduce the contact area directly contacting an inner surface of the outer member during and/or after introduction of the inner member into the outer member. Accordingly, manufacturing is eased during introduction process and rotational and translational movement of the inner member within the outer member during screwing the fixation member at the distal end of the lead. For example, in one embodiment of the manufacturing method, the profile comprises a plurality of alternating elevations and depressions, wherein the elevations and depressions extend along at least a part of the length of the inner member. A back line of such elevation or a ground line of such depression may extend parallel or helical with regard to the longitudinal axis of the inner member. In another embodiment, the profile comprises at least one helical elevation (i.e. without depression) extending along at least a part of the outer surface of the insulating layer. In both cases, the elevation may have a height between 5 and 30 mm, in particular a height of 15 mm (measured in radial direction with regard to the inner member). Additionally, if there are at least two neighboring elevations, their peak distance may be between 2 and 20 mm, in particular 10 mm in circumferential direction. The outer diameter of the wire spiral may be, for example, between 8 and 20 mm, in particular 12 mm, the outer diameter of the inner member (including the profile, if applicable) may be, for example, between 2 and 7 mm, in particular 4 mm. The material of the profile may correspond to the material of the electrically insulating layer or may be different. If the material of the profile and the electrically insulating layer is the same, the layer and its outer profile may be produced in a single application process.

In one embodiment of the manufacturing method, the method further comprises the step of attaching the terminal pin at a proximal end of the inner member such that the terminal pin is non-rotatably connected to the electrically insulating layer and the wire spiral as well as electrically connected to the wire spiral. The terminal pin may be attached, for example by adhesive bonding, welding or any similar permanent method. For example, the extruded electrically insulating layer may form a protruding collar at the proximal end of the wire spiral (or collar shaped proximal end section) and the terminal pin is attached to the inner member such that the collar (or the collar shaped section) surrounds the terminal pin at its distal end section thereby providing a reliably fixed connection. At the same time, the terminal pin forms a directly electrically conducting connection to the conducting part of the wire spiral. Basically, the terminal pin has the form of a cylinder and comprises at least one material of the group comprising metal, plastic or ceramic.

In one embodiment of the manufacturing method, the method further comprises the step of attaching the fixation helix at a distal end of the inner member such that the fixation helix is non-rotatably connected to the electrically insulating layer and the wire spiral as well as electrically connected to the wire spiral. The fixation helix may be attached, for example by adhesive bonding, welding or any similar permanent method. For example, the extruded electrically insulating layer may form a protruding collar at the distal end of the wire spiral (or a collar shaped distal end section) and the fixation helix is attached to the inner member such that the collar (or the collar shaped section) surrounds the fixation helix at its proximal end section thereby providing a reliably fixed connection. At the same time, the fixation helix forms a directly electrically conducting connection with the conducting part of the wire spiral. For attachment to the inner member the fixation helix may comprise a shaft section at its proximal end, wherein the dimensions of the shaft section are adapted to fit into the collar formed by the electrically insulating layer. The fixation helix may comprise a sharpened tip at its distal end section to ease penetration of the fixation helix into the tissue at the pre-defined location. The fixation helix may comprise any biocompatible and electrically conducting material, for example a material of the group comprising gold, aluminum and/ stainless steel.

The above problem is further solved by an implantable lead extending between a proximal end and a distal end and having an inner member, an outer tubular member, a terminal pin at the proximal end and a fixation helix at the distal end, wherein the outer tubular member comprises an electric conductor and the inner member is arranged between the terminal pin and the fixation helix as well as within the outer tubular member, wherein the inner member comprises an electrically conducting wire spiral that is fully covered at its exterior surface by an electrically insulating layer, the electrically insulating layer may, for example, be formed by extrusion onto the wire spiral of the inner member, wherein the electrically insulating layer comprises a profile at its outer surface configured to reduce the surface contact area with an inner surface of the outer tubular member. This measure further increases torque transmission from the proximal end to the distal end of the inner member thereby allowing better movement control of the fixation helix.

With regard to the elements of the implantable lead it is referred to the explanation above in connection with the manufacturing method. As indicated above the electrically insulating layer may be applied by extrusion onto the exterior surface of the wire spiral.

As indicated above, the profile at the outer surface of the electrically insulating may be provided by extrusion during application of the whole electrically insulating layer or by extrusion during application of the partial electrically insulating layer. The feature, that the profile at the outer surface of the electrically insulating layer is configured to reduce the surface contact area with the inner surface of the outer tubular member means that the surface contact area of the profile is lower than the surface contact area of a shell surface of a cylinder with the inner surface of the outer tubular member because the simplest contact area of the extruded (or otherwise applied) electrically insulating layer would be the cylinder-like shell surface. Embodiments of such profiles are described above.

As indicated above, in one embodiment of the lead the terminal pin is non-rotatably connected to the electrically insulating layer at the proximal end of the inner member and the wire spiral as well as electrically connected to the wire spiral.

Furthermore, in one embodiment of the lead the fixation helix is non-rotatably connected to the electrically insulating layer and the wire spiral at the distal end of the inner member and the fixation helix is electrically connected to the wire spiral.

In one embodiment of the lead the outer tubular member comprises a head portion at its distal end covering the fixation helix in an initial state, wherein the head portion comprises a guiding element, e.g. a thread section, cooperating with the fixation helix and forcing the fixation helix out of the head portion such that the fixation helix protrudes from the head portion if the inner member is rotated in a pre-defined direction. The head portion may be formed as a hollow cylinder. The head portion may be provided at the distal end of the outer member and fixedly connected to the outer member. In the initial state the head portion may fully cover and accommodate the fixation helix for protection. The head portion avoids injury of the patient's tissue by the fixation helix, for example during insertion of the lead into the patient's body.

The above problem is further solved by an implantable cardiac system comprising a control device (e.g. an artificial pacemaker, a defibrillator, a monitoring device) and at least one of above-described leads and/or at least one lead manufactured by an above-described method, wherein the respective lead is configured to be coupled to the control device. If the control device is an artificial pacemaker or a defibrillator, the control unit comprises a pulse generator. Further, the control device may comprise a battery forming a power source and/or a processor for signal analysis and control of pacing pulses. If the at least one lead is mechanically and electrically connected to the control device the electrically conducting wire spiral of the inner member of the at least one lead transmits or communicates the stimulation pulses and/or physiological (electric) signals between the heart and the control device.

The various features and advantages of the present invention may be more readily understood with reference to the following detailed description and the embodiments shown in the drawings. Herein schematically and exemplarily,
- Fig. 1: depicts a proximal end of a first embodiment of a lead in a longitudinal section,
- Fig. 2: shows a distal end of the embodiment of Fig. 1 in a longitudinal section,
- Fig. 3: shows a distal end of an inner member of a second embodiment of a lead in a perspective side view,
- Fig. 4: depicts a distal end of the second embodiment of a lead with the inner member of Fig. 3 in a perspective side view and in a partial longitudinal section,
- Fig. 5: shows an inner member of a third embodiment of a lead in a perspective side view, and
- Fig. 6: depicts an embodiment of an implantable medical system having two leads accommodated within a cross section of a heart.

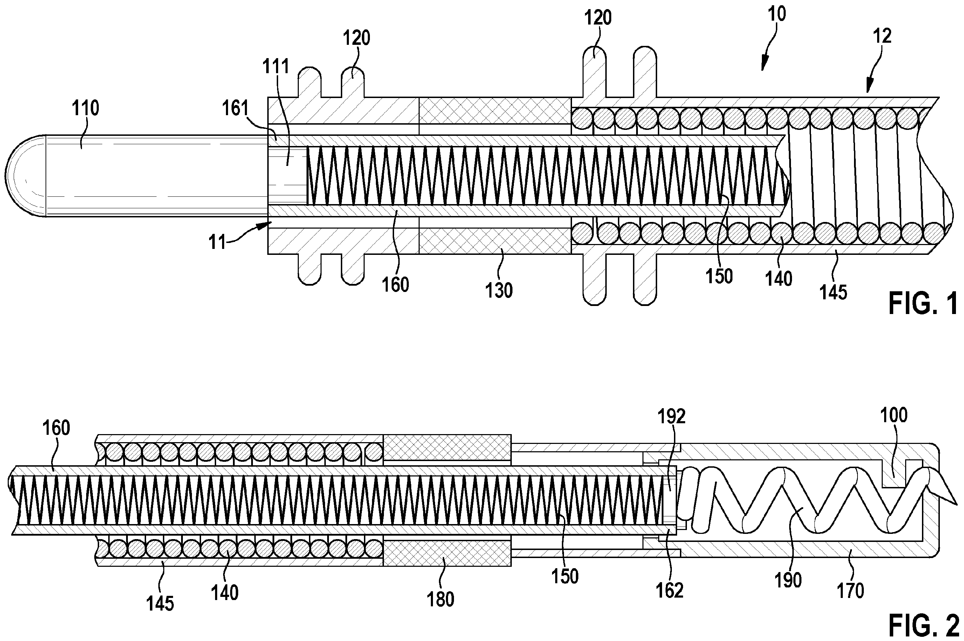

A first embodiment of an inventive lead 10 comprising an inner member 11 and a tubular outer member 12 is shown in Fig. 1 and 2, wherein the inner member 11 comprises a wire spiral 150 fully covered at its exterior surface by an electrically insulating layer 160. The inner member 11 is permanently and non-rotatably connected at its proximal end to the terminal pin 110 and at its distal end to the fixation helix 190. The outer member 12 comprises a wire spiral 140 and an electrically insulating and protecting sleeve 145 that covers the wire spiral 140 at its exterior surface. The outer member 12 further comprises sealing lips 120 at its proximal end as well as a first ring electrode 130 which is electrically connected to the wire spiral 140 of the outer member 12. At its distal end, the outer member comprises a second ring electrode 180 which is electrically connected to the wire spiral 140 of the outer member 12. The inner member 11 is accommodated within the inner lumen of the outer member 12.

Distal from the second ring electrode 180, the outer member is connected to a head section 170 formed as a hollow cylinder and configured to fully cover the fixation helix 190 in an initial state.

The terminal pin 110 is non-rotatably (fixedly) connected to the electrically insulating layer 160 and the wire spiral 150 of the inner member 11 so that a rotational movement of the terminal pin 110 provided by a user, e.g. a health care professional (HCP), is directly transmitted to the inner member 11 (including the electrically insulating layer 160). This is realized, for example by welding a shaft shaped distal end section 111 of the terminal pin 110 to a collar shaped proximal end section 161 of the electrically insulating layer 160 protruding from the proximal end of the wire spiral 150 of the inner member 11. The collar shaped proximal end section 161 encloses the distal end section 111 of the terminal pin 110 fully or at least over a considerable part of its circumference.

The fixation helix 190 is non-rotatably (fixedly) connected to the electrically insulating layer 160 and the wire spiral 150 of the inner member 11 so that a rotational movement of the terminal pin provided by a user, e.g. the HCP, is directly transmitted to the inner member 11 (including the electrically insulating layer 160) and further to the fixation helix 190. This is realized, for example, by the above-described non-rotatable connection of the terminal pin 110 and the inner member 11 and, for example, by welding a shaft shaped proximal end section 192 of the fixation helix 190 to a collar shaped distal end section 162 of the electrically insulating layer 160 protruding from the distal end of the wire spiral 150 of the inner member 11. The collar shaped proximal end section 162 encloses the proximal end section 192 of the fixation helix 190 fully or at least over a considerable part of its circumference. Accordingly, an improved transmission of the torque provided by the HCP at the terminal pin 110 to the fixation helix 190 is achieved.

The head section 170 comprises a thread section 100 cooperating with the fixation helix 190 if the terminal pin 110 is rotated into a pre-defined direction so that the fixation helix 190 is screwed out of the distal end of the head section 170 and protrudes therefrom or is screwed into the distal end of the head section 170 to withdraw the fixation helix 190, i.e. the fixation helix 190 executes a translational and rotational movement.

Regarding the manufacturing method, in one embodiment, the wire spiral 150 is provided and subsequently the electrically insulating layer 160 is extruded onto the exterior surface of the wire spiral 150, for example by using a nozzle. The material may be a thermoplastic material, for example PU. Herein, the electrically insulating layer 160 is extruded such that it forms a collar shaped proximal end section 161 protruding from the wire spiral 150 at its proximal end and a collar shaped distal end section 162 protruding from the wire spiral 150 at its distal end. This extrusion step is simple and may be automated by discretely or continuously introducing the wire spiral 150 into a respective extrusion facility. Accordingly, production costs of such lead are reduced.

In a subsequent step, the inner member 11 comprising the electrically insulating layer 160 and the wire spiral 150 is introduced into the outer member 12. Then, the terminal pin is welded to the collar shaped proximal end section 161 and the proximal end of the wire spiral 150 and the fixation helix 190 is welded to the collar shaped distal end section 162 and the distal end of the wire spiral 150 as described above. Afterwards, the head section 170 is fixed at the distal end of the outer member 12, for example by welding, thereby covering the fixation helix 190 in an initial state of the lead 10.

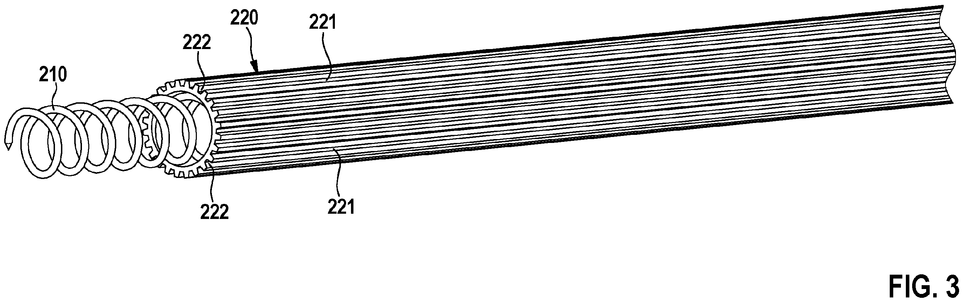

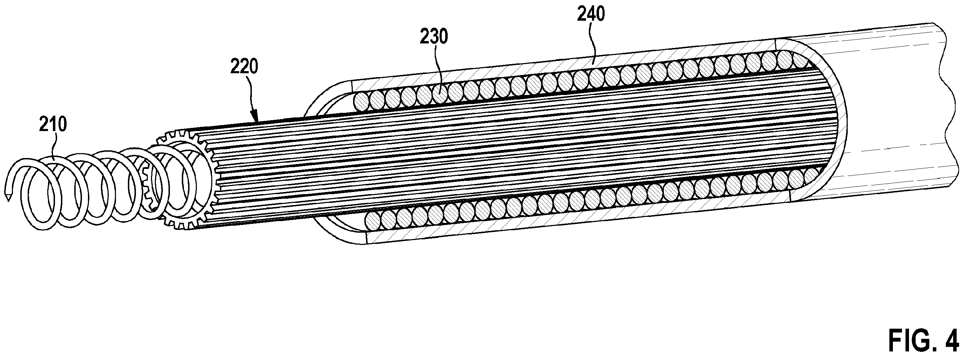

In the simplest version of the lead 10, the outer surface of the electrically insulating layer is formed generally as a shell surface of a cylinder. Alternatively, the outer surface may comprise a profile that decreases the contact surface area to the inner surface of the outer member 12. Such profile is shown at electrically insulating layer 220 of the inner member depicted in Fig. 3 and 4. The outer member comprises a wire spiral 230 and an outer sleeve 240 similar to the wire spiral 140 and the outer sleeve 145 of the outer member 12 shown in Fig. 1 and 2. The inner member is connected to a fixation helix 210 similar to the first embodiment. As one can derive from Fig. 3 and 4, the profile comprises a plurality of alternating elevations 221 and depressions 222, wherein the elevations and depression extend along the full length of the inner member / the electrically insulating layer 220 and parallel to the longitudinal axis of the inner member. The height of one elevation is, for example, 15 mm and the circumferential distance of two neighboring elevation peaks is, for example, 8 mm. The electrically insulating layer 220 contacts the inner surface of the wire spiral 230 of the outer member by the highest portion of the elevations 221 and hence with a smaller area compared to the shell surface of a cylinder. Accordingly, the friction between the inner and the outer member is reduced thereby further improving rotational and translational movement wherein, at the same time, the inner member is flexible.

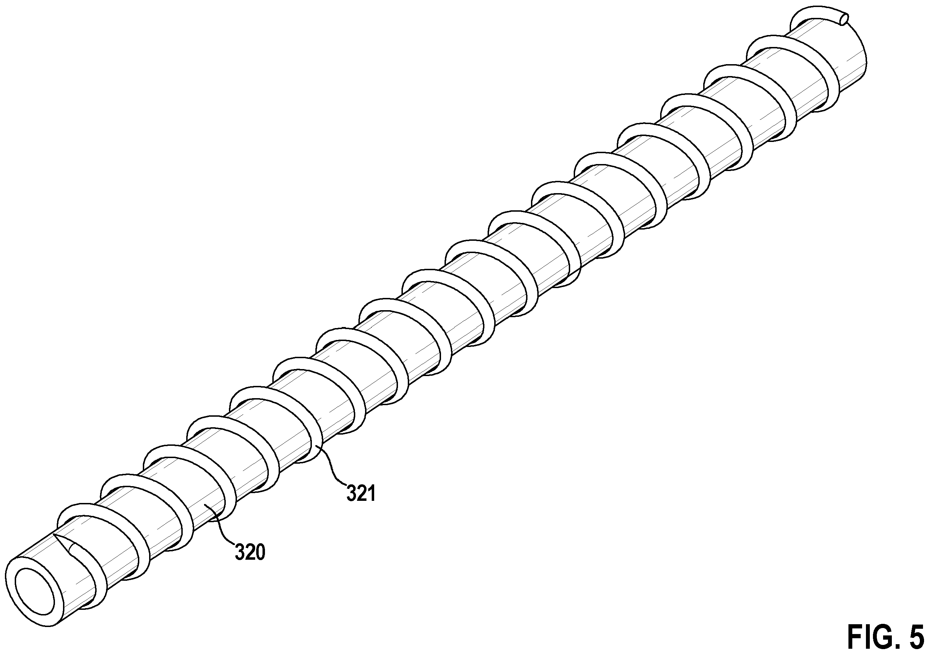

Alternatively, as depicted in Fig. 5, the profile at the outer surface of the electrically insulating layer 320 may be a helix shaped elevation 321 extending along almost the full length of the electrically insulating layer 320. The height of the elevation 321 is, for example, 1 mm and the distance of two neighboring windings of the elevation 321 in longitudinal direction is, for example, 3 mm.

The electrically insulating layers 220 and 320 depicted in Fig. 3 to 5 may be produced by extrusion as described with regard to the first embodiment.

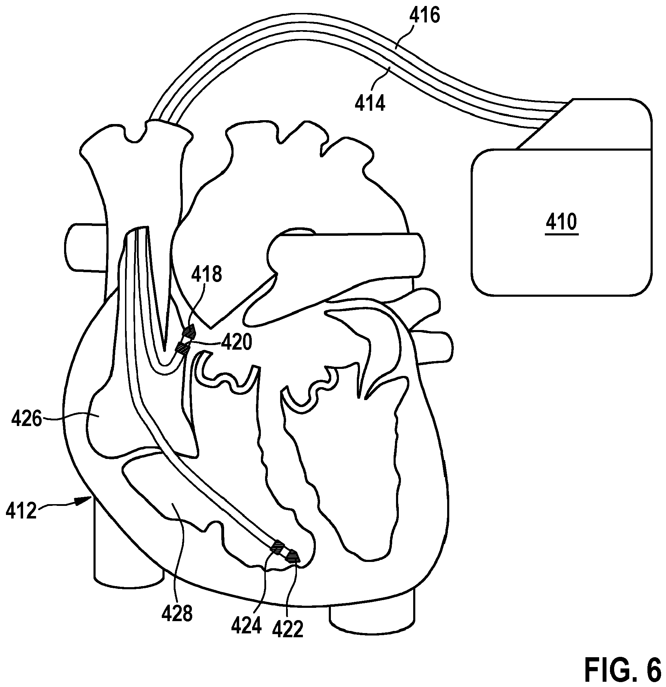

Finally, in Fig. 6 an inventive system in form of a dual chamber pacemaker 410 as heart stimulator connected to pacing/sensing leads placed in a heart 412 is illustrated. The pacemaker 410 is electrically coupled to heart 412 by way of leads 414 and 416. Lead 414 has a fixation helix 418 forming a tip electrode and a ring electrode 420 that are in contact with the right atrium 426 of the heart 412, wherein the lead 414 is similar to one of above explained embodiments of leads, e.g. the lead 10 (first embodiment) with the fixation helix 190 and the ring electrode 180. The same applies to lead 416 having a fixation helix 422 forming a tip electrode and a ring electrode 424 that are in contact with the right ventricle 428 of heart 412. During operation of the pacemaker leads 414 and 416 are connected to respective output/input terminals of pacemaker 410 as indicated in Fig. 6 with their proximal ends and transmit stimulating pulses to the fixation helices (electrodes) 418 and 422 from a pulse generator accommodated within the pacemaker 410. Further, electrical signals from the atrium 426 are carried from the electrode pair 418 and 420 through the lead 414 to a processor accommodated within the pacemaker 410 and electrical signals from the ventricle 428 are carried from the electrode pair 422 and 424 through the lead 416 to the processor of the pacemaker 410. The processor of the pacemaker 410 further comprises circuitry for timing ventricular and/or atrial stimulation pulses according to an adequate stimulation rate that can be adapted to a patient's hemodynamic needs. The pacemaker 410 may further comprise a battery forming the power source of the system.

## Claims

1. A manufacturing method for an implantable lead (10, 416, 417) extending between a proximal end and a distal end and having an inner member (11, 220, 320), an outer tubular member (12), a terminal pin (110) at the proximal end and a fixation helix (190, 418, 422) at the distal end, wherein the outer tubular member comprises an electric conductor (140, 230) and the inner member is arranged between the terminal pin and the fixation helix, wherein the inner member comprises an electrically conducting wire spiral (150), wherein the method comprises the following steps: providing the electrically conducting wire spiral (150) and extruding an electrically insulating layer (160, 220, 320) onto the conducting wire spiral and afterwards inserting the inner member into the outer tubular member.

2. The method of claim 1, wherein the extrusion is provided such that a profile (221, 222, 321) is created at the outer surface of the electrically insulating layer (220, 320).

3. The method of claim 2, wherein the profile comprises a plurality of alternating elevations (221) and depressions (222), wherein the elevations and depressions extend along at least a part of the length of the inner member (220).

4. The method of claim 2, wherein the profile comprises at least one helical elevation (321) extending along at least a part of the outer surface of the insulating layer (320).

5. The method of any one of the previous claims, wherein the method further comprises the step of attaching the terminal pin (110) at a proximal end of the inner member (11) such that the terminal pin is non-rotatably connected to the electrically insulating layer (160) and the wire spiral (150) as well as electrically connected to the wire spiral.

6. The method of any one of the previous claims, wherein the method further comprises the step of attaching the fixation helix (190) at a distal end of the inner member (11) such that the fixation helix is non-rotatably connected to the electrically insulating layer (160) and the wire spiral (150) as well as electrically connected to the wire spiral.

7. An implantable lead (10) extending between a proximal end and a distal end and having an inner member (11, 220, 320), an outer tubular member (12), a terminal pin (110) at the proximal end and a fixation helix (190) at the distal end, wherein the outer tubular member comprises an electric conductor (140, 230) and the inner member is arranged between the terminal pin and the fixation helix as well as within the outer tubular member, wherein the inner member comprises an electrically conducting wire spiral (150) that is fully covered at its exterior surface by an electrically insulating layer (160, 220, 320) formed by extrusion onto the wire spiral, wherein the electrically insulating layer comprises a profile (221, 222, 321) at its outer surface configured to reduce the surface contact area with an inner surface of the outer tubular member.

8. The lead of claim 7, wherein the terminal pin (110) is non-rotatably connected to the electrically insulating layer (160) at the proximal end of the inner member (11) and the wire spiral (150) as well as electrically connected to the wire spiral (150).

9. The lead of any one of claims 7 to 8, the fixation helix (190) is non-rotatably connected to the electrically insulating layer (160) and the wire spiral (150) at the distal end of the inner member (11) and the fixation helix (190) is electrically connected to the wire spiral (150).

10. The lead of any one of the claims 7 to 9, wherein the outer tubular member comprises a head portion (170) at its distal end covering the fixation helix (190) in an initial state, wherein the head portion comprises a guiding element (100) cooperating with the fixation helix and forcing the fixation helix out of the head portion such that the fixation helix protrudes from the head portion if the inner member (11) is rotated in a pre-defined direction.

11. The lead of any one of the claims 7 to 10, wherein the profile comprises a plurality of alternating elevations (221) and depressions (222), wherein the elevations and depressions extend along at least a part of the length of the inner member (220).

12. The lead of any one of the claims 7 to 10, wherein the profile comprises at least one helical elevation (321) extending along at least a part of the outer surface of the insulating layer (320).

13. An implantable cardiac system comprising a control device (410) and at least one lead (10, 414, 416) of any one of the claims 7 to 12 or at least one lead (10, 414, 416) manufactured by a method of any one of the claims 1 to 6, wherein the respective lead is configured to be coupled to the control device.
